# EUROPEAN PATENT SPECIFICATION

(11) **EP 3 813 565 B1**
(45) Date of publication and mention of the grant of the patent: **25.03.2026**
(21) Application number: 19734771.9
(22) Date of filing: 28.06.2019
(51) Int. Cl.: A24C 5/01, A24D 1/20, A61K 36/81, A24B 15/12, A24B 15/24

(54) **A METHOD FOR MANUFACTURING RECONSTITUTED PLANT MATERIAL**
VERFAHREN ZUR HERSTELLUNG VON REKONSTITUIERTEM PFLANZENMATERIAL
PROCÉDÉ DE FABRICATION DE MATÉRIAU VÉGÉTAL RECONSTITUÉ

(30) Priority: 29.06.2018 GB 201810729
(43) Date of publication of application: 05.05.2021
(73) Proprietor: Imperial Tobacco Limited, Bristol, BS3 2LL (GB)
(72) Inventor: FERRIE, Kate, Liverpool Merseyside L24 9HP (GB); SHENTON, Edward Ross, Liverpool Merseyside L24 9HP (GB)
(74) Representative: Haseltine Lake Kempner LLP
(86) International application number: PCT/EP2019/067304
(87) International publication number: WO 2020/002583

(56) References cited:
- WO-A1-2017/097840
- US-A- 3 385 303
- US-A- 5 143 097
- US-A- 5 377 698
- US-A1- 2017 055 566
- US-A1- 2017 273 358

## Description

### Field of the Invention

The present invention relates to a method for reconstituting a plant material by a paper-making process, and a reconstituted tobacco product.

### Background

During the manufacturing process for tobacco products, such as cigarettes, tobacco waste is produced. The tobacco waste is typically in the form of tobacco dust, leaf or stem. The tobacco waste can be collected and processed to provide what is known as reconstituted tobacco, or "recon".

Two distinct methods are most commonly employed to manufacture reconstituted tobacco, these are i) a paper-making process, and ii) slurry casting. The products that result from these distinct processes have notably different properties and characteristics. In particular, recon formed by the paper-making process typically has a reduced aroma/flavour and nicotine content in comparison to that formed by slurry casting.

In slurry casting the tobacco waste is milled to a fine powder and mixed with an aqueous solvent, typically water. The resultant slurry may undergo further milling to reduce the particle size of the tobacco material further. The slurry is then cast on a surface and dried to form a sheet. The dried sheet may be shredded to be used within various tobacco products, for example as a cigarette filler.

In the paper-making process the tobacco waste is mechanically beaten in the presence of an aqueous solvent to digest and process the tobacco into workable fibres to be made into a paper or web. Subsequently, water soluble compounds present in the tobacco are extracted into the aqueous solvent. The aqueous extract and insoluble fibrous portion are separated. The separated fibrous portion, which may also be described as a "pulp", undergoes further processing to form a base sheet via a typical paper-making procedure. The aqueous extract is concentrated and then reapplied to the base sheet. The base sheet is then dried to form a paper.

The paper formed by the above process can be shredded and used as a filler within typical smoking articles such as cigarettes. Alternatively, it may be used as a wrapper to contain a tobacco rod. Conventional reconstituted tobacco paper of this kind is generally not suitable for "heat-not-burn" ("HNB") devices. For recon paper to be suitable for use within a HNB device a humectant must also be incorporated. Typical humectants include propylene glycol ("PG") or vegetable glycerine ("VG").

An HNB device heats the tobacco without burning it, i.e. it does not undergo combustion. The purpose of HNB devices is to avoid production, and subsequent inhalation, of harmful combustion products such as tar. By heating a humectant-impregnated recon paper the humectant is vaporised to form an aerosol, also referred to as a "vapour". The aerosol may contain aroma/flavour compounds and/or nicotine derived from the recon paper. It is the aerosol which enables the user to simulate the act of smoking. WO 2017/051034 describes a reconstituted tobacco made by a paper making process which is suitable for an HNB device. In which, a humectant is added to the concentrated tobacco extract before application to the base-sheet to provide a humectant-impregnated recon paper.

Associated with the recon paper-making process is the loss of volatile compounds, such as nicotine and flavour compounds, from the tobacco during processing. This is linked with the large volumes of water used to digest and process the tobacco, and the high temperatures used to evaporate the water.

It is desirable to provide a safe and efficient way of increasing the nicotine and/or flavour content of recon tobacco made by a paper-making process. In particular it is desirable to provide a product that delivers an enhanced sensory experience to the consumer, which is consistent over multiple consumables.

Various efforts have been made to improve the nicotine content of recon tobacco made by a paper-making process. In US 3422819, the nicotine content of a cigarette wrapper was increased by applying a solution of nicotine salt to a reconstituted tobacco sheet after manufacture of the paper. Alternatively, it is recognised in US 3422819 that "the solution of nicotine salt may be added to the stock from which paper is formed". The term "stock" refers to the mixture of pulp, fillers, other papermaking materials and water (see C. Biermann, Handbook of Pulping and Papermaking, 2nd Edition). The stock, therefore, is the fibrous mixture post-extraction that enters the paper-making machine to be formed into a base sheet.

US 3,861,400 describes a method of increasing the nicotine content of recon tobacco formed by a slurry casting method. This is achieved by the addition of specific polyuronic acid nicotine salt derivatives to a tobacco-water slurry. The products produced by slurry casting, and the method itself, are distinct to that of the recon paper-making process.

GB 1466912 describes an alternative method of increasing the nicotine content within recon tobacco paper. In which, roots of plants of the genus *Nicotiana* are grown in a specific culture medium. The grown roots are combined with tobacco solids to form a reconstituted tobacco base sheet. The spent culture medium, which includes nicotine exuded by the roots, is added to the tobacco soluble obtained in the production of the reconstituted tobacco. The tobacco soluble is concentrated and added to the base sheet.

CN 103468403 and CN 103462204 describe a method for extracting tobacco aroma compounds by conducting two separate extraction processes directly upon the tobacco raw material. This includes separate water extraction and supercritical carbon dioxide (scCO₂) extraction on the tobacco raw material. The two extracts are then combined and applied to the reconstituted tobacco. Alternatively, CN 103783660 describes scCO₂ extraction on tobacco leaf fragments to extract flavour compounds. The flavour compounds are then separated, purified and reapplied to a reconstituted tobacco product by spraying or coating.

In the context of reconstituted tobacco suitable for use within a HNB device a number of documents describe that flavour additives may be optionally added to the humectant-impregnated recon paper, see for example, WO2017/051034, US 5325877, US 5715844 and US 2006/0021626. However, it is not specified within these documents how the flavour additives are added, nor at what stage in the paper-making process the flavour additives are added. Indeed, it is not recognised that flavour compounds are lost during the recon paper-making process. Therefore, it is apparent that the flavour additives are not intended to replace lost flavour during processing, but instead to impart a different taste experience to that of tobacco.

US3385303 concerns a slurry casting method of preparing a tobacco product from bright tobacco stems, by treating the bright tobacco stems to remove the calcium and magnesium crosslinks from the pectinaceous material contained therein. US2017/055566 concerns approaches to modify the expression of a gene that is involved in the production of a harmful compound in tobacco. WO2017097840 concerns a tobacco composition comprising a tobacco component in an amount of from 60 to 90% by weight of the tobacco composition, a filler component in an amount of 0 to 20% by weight of the tobacco composition, and an aerosol generating agent in an amount of from 10 to 20% by weight of the tobacco composition; wherein the tobacco composition has a nicotine content of from 0.5 to 1.5% by weight of the tobacco composition; and wherein the tobacco component comprises paper reconstituted tobacco in an amount of from 70 to 100% by weight of the tobacco component. The present invention has been devised in light of the above considerations.

### Summary of the Invention

According to a first aspect of the invention, there is provided a method for manufacturing a reconstituted plant material, the method comprising:
extracting a fibrous plant material with a solvent to provide an extract and a fibrous portion;
separating the extract from the fibrous portion;
forming a base sheet from the fibrous portion;
combining a nicotine salt with the extract to provide a modified extract;
applying the modified extract to the base sheet.

Nicotine is a highly volatile compound and a controlled substance, consequently a number of handling difficulties exist during processing, as it is the subject of strict exposure limits. Similarly, many flavour compounds are volatile and are also the subject of exposure limits.

Advantageously, nicotine salts exhibit lower volatility and higher stability than nicotine, therefore provide a safer and more convenient means of handling during processing. Additionally, by including a nicotine salt-addition step in-situ to the paper-making process, nicotine losses during processing are negated (e.g. losses through evaporation and high dilution). Furthermore, combining a nicotine salt with the extract ensures improved impregnation and uniform distribution of the nicotine salt throughout the final product. This provides a more consistent and improved sensory experience to the user.

Also, by providing the nicotine salt to the extract (which also contains other soluble compounds such as flavour compounds derived from the plant material) a homogeneous mixture of nicotine salt and flavour compounds is obtained. Consequently, a greater degree of intermingling of nicotine salt and flavour compounds is achieved in the recon paper to provide an improved sensory experience to the user.

During extraction the mixture of fibrous plant material and solvent are mechanically beaten to form a pulp. A pulp consists of lignocellulosic materials, e.g. plant fibres, which have been broken down physically and/or chemically such that discrete fibres are liberated and can be dispersed in solvent and reformed to form a web. Optionally, additional chemicals may be added to the mixture to aid digestion of the tobacco material and pulp formation. Suitable chemicals are known to those skilled in the art.

Suitable solvents are known to those skilled in the art. These include non-aqueous solvents and aqueous solvents. Examples of non-aqueous solvents includes but is not limited to alcohols, such as ethanol and propanol; acetone; acetonitrile; benzene; dichloromethane; ethyl acetate; hexane; and toluene.

Preferably, the solvent is an aqueous solvent. More preferably, the solvent is water. Optionally, the aqueous solvent may contain a co-solvent. Preferably, the co-solvent is water miscible. Examples of co-solvents are alcohols, acetone and acetonitrile.

Advantageously, water is a non-toxic and non-flammable solvent and is thus is easier to handle during processing. Furthermore, the risk of toxic solvent residues remaining in the final product is removed.

The temperature of the tobacco-water mixture during extraction is generally within the range 10 - 100 °C. Preferably, the temperature is within the range 30 - 90 °C. Particularly, preferred is the range 30 - 70 °C.

The extraction time generally ranges from 30 minutes to 6 hours. Preferably, the extraction time is less than 4 hours, such as less than 3 hours, or such as less than 2 hours. Particularly, preferred is an extraction time less than 1 hour.

Preferably, the method further comprises a step of concentrating the extract to provide a concentrated extract, before applying the extract to the base sheet.

Advantageously, this minimises the drying time of the base sheet and improves the process efficiency of applying the extract to the paper.

Preferably, the nicotine salt is combined with the concentrated extract.

Advantageously, the combination of the nicotine salt with the concentrated extract minimises losses of the nicotine salt through decomposition and/or evaporation during concentration of the extract.

Preferably, the fibrous plant material is tobacco.

Advantageously, as the invention is primarily intended to provide a smoking consumable, the use of tobacco provides a reconstituted plant material product having the inherent properties of tobacco, for example the texture, aroma and nicotine content.

Preferably, the tobacco is in the form of tobacco leaf, tobacco stem, tobacco powder and/or tobacco dust.

Advantageously, these forms of tobacco are typically derived as waste from tobacco processing, thus by utilising this "waste" to form the product of the invention the amount of unused tobacco material is minimised.

Preferably, the nicotine salt is selected from nicotine hydrochloride, nicotine dihydrochloride, nicotine monotartrate, nicotine bitartrate, nicotine bitartrate dihydrate, nicotine sulphate, nicotine zinc chloride monohydrate and nicotine salicylate, and combinations thereof.

Advantageously, these salts provide the user with a satisfactory nicotine "hit" and additionally provide an improved sensory experience to the user.

Preferably, the nicotine salt is provided as an aqueous solution.

Advantageously, the non-toxicity and non-flammability of water minimises handling risks during processing. Furthermore, the "premixing" of the nicotine salt with a solvent prior to combination with the extract, improves the homogeneous nature of the nicotine salt and extract mixture.

Preferably, the aqueous solution of nicotine salt includes a co-solvent, for example alcohols, acetone or acetonitrile

Advantageously, the presence of a co-solvent may improve the solubility of the nicotine salt in solution. This may help improve the homogeneity of the mixture of the nicotine salt solution and the extract.

The method according to the first aspect of the invention may further comprise a step of making an HNB consumable.

According to a second aspect of the invention, there is provided a reconstituted tobacco product for an HNB device produced by a process according to the first aspect of invention comprising:
a nicotine salt selected from nicotine hydrochloride, nicotine dihydrochloride, nicotine monotartrate, nicotine bitartrate, nicotine bitartrate dihydrate, nicotine sulphate, nicotine zinc chloride monohydrate and nicotine salicylate, and combinations thereof,
wherein the nicotine content is 2 to 25 % by weight of the reconstituted tobacco product.

Advantageously, by increasing the minimum amount of nicotine present in the reconstituted tobacco product a stronger nicotine "hit" may be experienced by the user. In addition, the size of the product itself may be decreased, in comparison to known recon paper products, without any reduction in the total amount of nicotine delivered to the user per consumable.

The nicotine content of the reconstituted tobacco product has a lower limit of at least 2 wt % of the weight of the reconstituted tobacco product, such as at least 3 wt %, such as at least 4 wt %, such as at least 5 wt %, such as at least 10 wt %, such as at least 15 wt %, such as at least 20 wt %.

The nicotine content of the reconstituted tobacco product has an upper limit of at most 25 wt %, such as at most 20 wt %, such as at most 15 wt %, or such as at most 10 wt %.

Preferably, the nicotine content is 5 to 25 % by weight of the reconstituted tobacco product, such as 10 to 25 wt %, such as 15 to 25 wt %.

Advantageously, a reconstituted tobacco product with nicotine content higher than achieved using typical processes is provided. This enables the amount of nicotine to be tailored to suit the user's requirements.

According to a third aspect of the invention, there is provided use of the reconstituted tobacco product according to the second aspect of the invention in a HNB device.

According to a fourth aspect of the invention, there is provided an HNB consumable comprising the reconstituted tobacco product according to the second aspect of the invention.

According to a fifth aspect of the invention, there is provided an HNB system comprising an HNB consumable according to the fourth aspect of the invention, and an HNB device.

According to a sixth aspect, there is provided a provided a reconstituted tobacco product comprising:
a nicotine salt selected from nicotine hydrochloride, nicotine dihydrochloride, nicotine monotartrate, nicotine bitartrate, nicotine bitartrate dihydrate, nicotine sulphate, nicotine zinc chloride monohydrate and nicotine salicylate, and combinations thereof,
wherein the nicotine content is 2 to 25 % by weight of the reconstituted tobacco product.

### Brief Description of the Drawings

A complete understanding of preferred embodiments of the present invention may be obtained by reference to the accompanying drawings, when considered in conjunction with the subsequent, detailed description, provided by way of example only and in which:

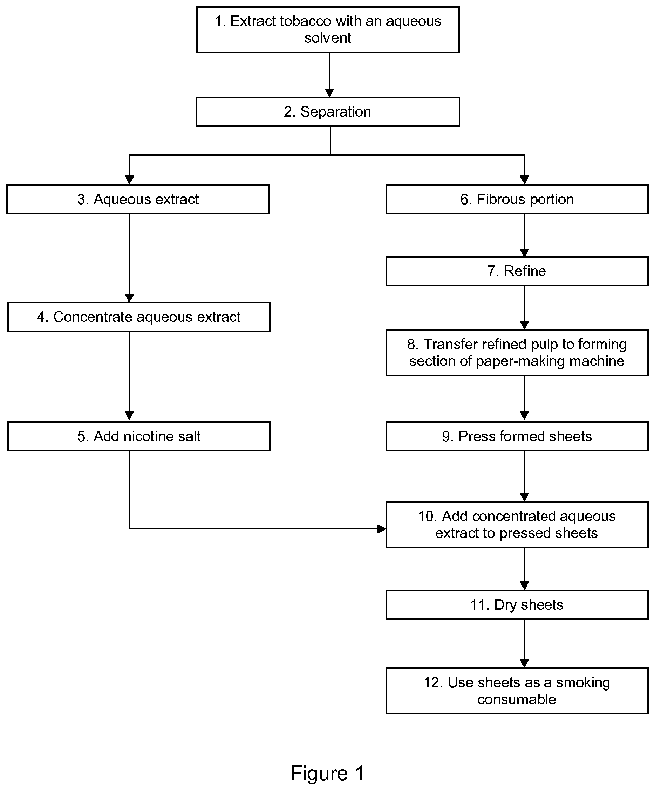

Figure 1 is an illustrative view of a manufacturing process for producing reconstituted tobacco for use in accordance with the present invention

### Detailed Description of the Invention

It is to be understood that the terminology used herein is for the purpose of describing particular embodiments only, and not intended to be limiting, since the scope of the present invention will be limited by the appended claims.

Unless defined otherwise, all technical and scientific terms used herein have the same meaning as commonly understood by one of ordinary skill in the art to which this invention belongs or as determined by the context in which such terms are used. Although any methods and materials similar or equivalent to those described herein can also be used in the practise or testing of the present invention, a limited number of exemplary methods and materials are described herein.

The publications discussed herein are provided solely for their disclosure prior to the filing date of the present patent application. The present invention provides a method for manufacturing reconstituted plant material comprising the following steps:
extracting a fibrous plant material with a solvent to provide an extract and a fibrous portion;
separating the extract from the fibrous portion;
forming a base sheet from the fibrous portion;
combining a nicotine salt with the extract to provide a modified extract;
applying the modified extract to the base sheet.

Figure 1 illustrates the preferred embodiment of the invention, wherein the fibrous plant material is tobacco, and wherein the nicotine salt is combined with a concentrated aqueous extract. With reference to Figure 1 this embodiment is described in detail below.

Any suitable plant material that requires reconstituting as a paper, wherein the loss of volatile compounds during processing (such as alkaloid derivatives or flavour compounds) is detrimental to the final product, may be used in the present invention.

Preferably, the plant material is tobacco. Any type of tobacco may be used in the present invention. This includes, but is not limited to, flue-cured tobacco, burley tobacco, Maryland Tobacco, dark-air cured tobacco, oriental tobacco, dark-fired tobacco, perique tobacco and rustica tobacco. This also includes blends of the above mentioned tobaccos.

Any suitable parts of the tobacco plant may be used. This includes leaves, stems, roots, bark, seeds and flowers. In particular, tobacco waste is used, tobacco waste is typically in the form of tobacco leaf, stem, powder or dust.

Initially, the tobacco material is added to an aqueous solvent to extract the water soluble tobacco components **1.** Prior to this the tobacco material may be optionally grinded, threshed or cut.

Typically, the amount of aqueous solvent to tobacco material is within the range 75 - 99 wt % of the total weight of the tobacco-aqueous solvent mixture.

The tobacco-aqueous solvent mixture is mechanically beaten during extraction **1** in order to create a pulp.

After extraction **1,** the fibrous portion and aqueous extract are separated, step **2.** This may be achieved by centrifugation, filtration, mechanical pressing or any other methods known in the paper-making industry.

The separated fibrous portion **6** is further refined to form a refined pulp suitable for entry into the paper-making machine **7.** Typical pulp refiners include disk refiners and conical refiners. The refined pulp is then properly slurried and treated to remove contaminants and entrained air.

The refined pulp slurry is applied to a paper-making machine **8.** The paper-making machine comprises a forming section **8,** drainage section, pressing section **9** and a drying section **11.** In the forming section **8** the slurry of refined pulp **7** is applied to a wire sheet, also known as a "forming fabric", to form a web. The wire permits draining of excess water from the web by gravity to form a sheet. Alternatively, suction equipment may also be used permit drainage. Excess water is further removed by means of a press **9.** The press is a typically a pair of squeeze or wringer rolls designed to remove water mechanically and smooth and compress the sheet formed. Drying **11** of the sheet is typically facilitated by drum dryers.

The aqueous extract **3** is concentrated **4.** This is achieved by any conventional means, for example by applying thermal energy and/or evaporation under vacuum.

A nicotine salt is combined with the concentrated aqueous extract in step **5.** The nicotine salt may be combined in its pure form. The nicotine salt may be a solid or a liquid. Alternatively, the nicotine salt may be an aqueous solution. The amount of nicotine combined may be tailored to the content required in the final product.

The concentrated aqueous extract **5** is preferably applied to the sheet after the sheet has proceeded through the pressing section, and before the sheet has proceeded through the drying section of the paper-making machine, step **10.** The concentrated aqueous extract **5** may be applied to the sheet using spray coating, wetted rollers or any other method suitable in the art. Following this the sheet is dried **11.**

The present invention also provides a reconstituted tobacco product for an HNB device produced by a process according to the method of the invention, wherein the reconstituted tobacco product comprises:
a nicotine salt selected from nicotine hydrochloride, nicotine dihydrochloride, nicotine monotartrate, nicotine bitartrate, nicotine bitartrate dihydrate, nicotine sulphate, nicotine zinc chloride monohydrate and nicotine salicylate, and combinations thereof, and wherein the nicotine content is 2 to 25 % by weight of the reconstituted tobacco product .

The reconstituted tobacco product of the present invention may be used as a tobacco rod, suitable to be ensleeved by a wrapper such as a cigarette paper **12.** The reconstituted tobacco product may be shredded to form a tobacco rod. Alternatively, the reconstituted tobacco product may be wrapped or folded to form a tobacco rod **12.**

The reconstituted tobacco product of the present invention may also be used as a consumable, or part of a consumable, for an HNB device. It is a reconstituted tobacco product for use in an HNB device.

For the avoidance of any doubt, any theoretical explanations provided herein are provided for the purposes of improving the understanding of a reader. The inventors do not wish to be bound by any of these theoretical explanations.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

## Claims

1. A method for manufacturing a reconstituted plant material comprising the following steps:
extracting a fibrous plant material with a solvent to provide an extract and a fibrous portion;
separating the extract from the fibrous portion;
forming a base sheet from the fibrous portion;
combining a nicotine salt with the extract to provide a modified extract;
applying the modified extract to the base sheet.

2. The method according to claim 1, wherein the solvent is an aqueous solvent.

3. The method according to claim 1 or claim 2, comprising a step of concentrating the extract to provide a concentrated extract, wherein the nicotine salt is combined with the concentrated extract to provide a concentrated modified extract.

4. The method according to any one of claims 1 to 3, wherein the fibrous plant material is tobacco.

5. The method according to claim 4, wherein the tobacco is in the form of tobacco leaf, tobacco stem, tobacco powder, and/or tobacco dust.

6. The method according to any one of claims 1 to 5, wherein the nicotine salt is selected from nicotine hydrochloride, nicotine dihydrochloride, nicotine monotartrate, nicotine bitartrate, nicotine bitartrate dihydrate, nicotine sulphate, nicotine zinc chloride monohydrate and nicotine salicylate, and combinations thereof.

7. The method according to any one of claims 1 to 6, wherein the nicotine salt is provided as an aqueous solution.

8. The method according to claim 7, wherein the aqueous solution of nicotine salt comprises a co-solvent.

9. A reconstituted tobacco product for an HNB device produced by a process according to at least one of the preceding claims comprising:
a nicotine salt selected from nicotine hydrochloride, nicotine dihydrochloride, nicotine monotartrate, nicotine bitartrate, nicotine bitartrate dihydrate, nicotine sulphate, nicotine zinc chloride monohydrate and nicotine salicylate, and combinations thereof
wherein the nicotine content is 2 to 25 % by weight of the reconstituted tobacco product.

10. The reconstituted tobacco product according to claim 9, wherein the nicotine content is 5 to 25 % by weight of the reconstituted tobacco product, such as 10 to 25 wt %, such as 15 to 25 wt %.

11. Use of the reconstituted tobacco product according to claim 9 or claim 10 in a HNB device.

## Patentansprüche

1. Verfahren zur Herstellung eines rekonstituierten Pflanzenmaterials, das die folgenden Schritte umfasst:
Extrahieren eines faserigen Pflanzenmaterials mit einem Lösungsmittel, um einen Extrakt und einen faserigen Anteil bereitzustellen;
Trennen des Extrakts von dem faserigen Anteil;
Bilden einer Basisbahn aus dem faserigen Anteil;
Kombinieren eines Nikotinsalzes mit dem Extrakt, um einen modifizierten Extrakt bereitzustellen;
Aufbringen des modifizierten Extrakts auf die Basisbahn.

2. Verfahren nach Anspruch 1, wobei das Lösungsmittel ein wässriges Lösungsmittel ist.

3. Verfahren nach Anspruch 1 oder Anspruch 2, umfassend einen Schritt des Konzentrierens des Extrakts, um einen konzentrierten Extrakt bereitzustellen, wobei das Nikotinsalz mit dem konzentrierten Extrakt kombiniert wird, um einen konzentrierten modifizierten Extrakt bereitzustellen.

4. Verfahren nach einem der Ansprüche 1 bis 3, wobei das faserige Pflanzenmaterial Tabak ist.

5. Verfahren nach Anspruch 4, wobei der Tabak in Form von Tabakblatt, Tabakstängel, Tabakpulver und/oder Tabakstaub vorliegt.

6. Verfahren nach einem der Ansprüche 1 bis 5, wobei das Nikotinsalz ausgewählt ist aus Nikotinhydrochlorid, Nikotindihydrochlorid, Nikotinmonotartrat, Nikotinbitartrat, Nikotinbitartratdihydrat, Nikotinsulfat, Nikotinzinkchloridmonohydrat und Nikotinsalicylat und Kombinationen davon.

7. Verfahren nach einem der Ansprüche 1 bis 6, wobei das Nikotinsalz als eine wässrige Lösung bereitgestellt wird.

8. Verfahren nach Anspruch 7, wobei die wässrige Lösung von Nikotinsalz ein Co-Lösungsmittel umfasst.

9. Rekonstituiertes Tabakprodukt für eine HNB-Vorrichtung, hergestellt durch ein Verfahren nach mindestens einem der vorhergehenden Ansprüche, umfassend:
ein Nikotinsalz, ausgewählt aus Nikotinhydrochlorid, Nikotindihydrochlorid, Nikotinmonotartrat, Nikotinbitartrat, Nikotinbitartratdihydrat, Nikotinsulfat, Nikotinzinkchloridmonohydrat und Nikotinsalicylat und Kombinationen davon
wobei der Nikotingehalt 2 bis 25 Gew.-% des rekonstituierten Tabakprodukts beträgt.

10. Rekonstituiertes Tabakprodukt nach Anspruch 9, wobei der Nikotingehalt 5 bis 25 Gew.-% des rekonstituierten Tabakprodukts beträgt, wie etwa 10 bis 25 Gew.-%, wie etwa 15 bis 25 Gew.-%.

11. Verwendung des rekonstituierten Tabakprodukts nach Anspruch 9 oder Anspruch 10 in einer HNB-Vorrichtung.

## Revendications

1. Procédé de fabrication d'un matériau végétal reconstitué comprenant les étapes suivantes :
extraction d'un matériau végétal fibreux avec un solvant pour fournir un extrait et une fraction fibreuse ;
séparation de l'extrait de la partie fibreuse ;
formation d'une feuille de base à partir de la partie fibreuse ;
combinaison d'un sel de nicotine avec l'extrait pour fournir un extrait modifié ;
application de l'extrait modifié à la feuille de base.

2. Procédé selon la revendication 1, dans lequel le solvant est un solvant aqueux.

3. Procédé selon la revendication 1 ou la revendication 2, comprenant une étape de concentration de l'extrait pour fournir un extrait concentré, dans lequel le sel de nicotine est combiné avec l'extrait concentré pour fournir un extrait modifié concentré.

4. Procédé selon l'une quelconque des revendications 1 à 3, dans lequel le matériau végétal fibreux est du tabac.

5. Procédé selon la revendication 4, dans lequel le tabac se présente sous forme de feuille de tabac, de tige de tabac, de poudre de tabac et/ou de poussière de tabac.

6. Procédé selon l'une quelconque des revendications 1 à 5, dans lequel le sel de nicotine est choisi parmi le chlorhydrate de nicotine, le dichlorhydrate de nicotine, le monotartrate de nicotine, le bitartrate de nicotine, le bitartrate de nicotine dihydraté, le sulfate de nicotine, le chlorure de zinc monohydraté de nicotine et le salicylate de nicotine, et leurs combinaisons.

7. Procédé selon l'une quelconque des revendications 1 à 6, dans lequel le sel de nicotine est fourni sous forme d'une solution aqueuse.

8. Procédé selon la revendication 7, dans lequel la solution aqueuse de sel de nicotine comprend un co-solvant.

9. Produit de tabac reconstitué pour un dispositif HNB, produit par un processus selon au moins l'une des revendications précédentes comprenant :
un sel de nicotine choisi parmi le chlorhydrate de nicotine, le dichlorhydrate de nicotine, le monotartrate de nicotine, le bitartrate de nicotine, le bitartrate de nicotine dihydraté, le sulfate de nicotine, le chlorure de zinc monohydraté de nicotine et le salicylate de nicotine, et leurs combinaisons
dans lequel la teneur en nicotine est de 2 à 25 % en poids du produit de tabac reconstitué.

10. Produit de tabac reconstitué selon la revendication 9, dans lequel la teneur en nicotine est de 5 à 25 % en poids du produit de tabac reconstitué, par exemple de 10 à 25 % en poids, par exemple de 15 à 25 % en poids.

11. Utilisation du produit de tabac reconstitué selon la revendication 9 ou la revendication 10 dans un dispositif HNB.
